(19) 

Europäisches
Patentamt

European
Patent Office

Office européen
des brevets

(11) **EP 4 527 289 A1**

(12) **EUROPEAN PATENT APPLICATION**
published in accordance with Art. 153(4) EPC

(43) Date of publication:
26.03.2025 Bulletin 2025/13

(21) Application number: 24809197.7

(22) Date of filing: 24.06.2024

(51) International Patent Classification (IPC):
**A61B 5/022** (2006.01)     **A61B 5/00** (2006.01)

(86) International application number:
**PCT/CN2024/100910**

(87) International publication number:
**WO 2025/031036** (13.02.2025 Gazette 2025/07)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 10.08.2023 CN 202311010001

(71) Applicant: Huawei Technologies Co., Ltd.
Shenzhen, Guangdong 518129 (CN)

(72) Inventors:
• CHEN, Qing
Shenzhen, Guangdong 518129 (CN)

• HUANG, Yadong
Shenzhen, Guangdong 518129 (CN)
• WU, Jiangfei
Shenzhen, Guangdong 518129 (CN)
• WANG, Youhua
Shenzhen, Guangdong 518129 (CN)
• JIN, Junye
Shenzhen, Guangdong 518129 (CN)

(74) Representative: MERH-IP Matias Erny Reichl
Hoffmann
Patentanwälte PartG mbB
Paul-Heyse-Straße 29
80336 München (DE)

(54) **WEARING DETECTION METHOD FOR WEARABLE DEVICE, AND WEARABLE DEVICE**

(57)     This application provides a wearing detection method for a wearable device and the wearable device. The wearable device includes a main body, a wrist strap, and a blood pressure detection component. The blood pressure detection component includes an airbag and an air pump. The method includes: detecting that a preset condition is met, and inflating the airbag; detecting a volume expansion rate of the airbag; determining a wearing detection result of the wearable device based on the volume expansion rate; and displaying a first interface, where the first interface includes the wearing detection result. **In** embodiments of this application, the wearable device may determine, by detecting the volume expansion rate of the airbag, whether the wearable device is normally worn. This ensures accuracy of a physiological parameter measurement result, without adding hardware, and without incurring additional costs.

```
S601: Detect that a preset condition is met, and inflate an airbag

S602: Detect a volume expansion rate of the airbag within T seconds

S603: Determine a wearing detection result of a wearable device based on the volume
expansion rate of the airbag within the T seconds

S604: Display a first interface, where the first interface includes the wearing detection result
```

FIG. 6

## Description

This application claims priority to Chinese Patent Application No. 202311010001.X, filed with the China National Intellectual Property Administration on August 10, 2023 and entitled "WEARABLE DETECTION METHOD FOR WEARING DEVICE AND WEARABLE DEVICE", which is incorporated herein by reference in its entirety.

## TECHNICAL FIELD

Embodiments of this application relate to the field of wearable devices, and more specifically, to a wearing detection method for a wearable device and the wearable device.

## BACKGROUND

Nowadays, people are increasingly concerned about their own and family health conditions, and blood pressure measurement, heart rate measurement, blood oxygen measurement, and the like become particularly important. With progress and development of science and technology, functions such as blood pressure measurement, heart rate measurement, and blood oxygen measurement are also integrated into some wearable devices (such as smartwatches or smart bands), providing a user with a possibility of performing measurement anytime and anywhere. During such measurement, measurement accuracy is affected if the user wears the wearable device too tight or too loose. Therefore, how to detect whether the wearable device is normally worn becomes a technical problem to be urgently resolved.

## SUMMARY

This application provides a wearing detection method for a wearable device and the wearable device, to determine whether the wearable device is normally worn by detecting a volume expansion rate of an airbag. This ensures accuracy of a physiological parameter measurement result, without adding hardware, and without incurring additional costs.

According to a first aspect, a wearing detection method for a wearable device is provided. The wearable device includes a main body, a wrist strap, and a blood pressure detection component. The blood pressure detection component includes an airbag and an air pump. The method includes: detecting that a preset condition is met, and inflating the airbag, where an inflation time period is T seconds, and T>0; detecting a volume expansion rate of the airbag within the T seconds; determining a wearing detection result of the wearable device based on the volume expansion rate of the airbag within the T seconds; and displaying a first interface, where the first interface includes the wearing detection result.

In an embodiment of this application, the wearable device may determine, by detecting the volume expansion rate of the airbag, whether the wearable device is normally worn. This ensures accuracy of a physiological parameter measurement result, without adding hardware, and without incurring additional costs.

With reference to the first aspect, in some implementations of the first aspect, before the determining that a preset condition is met, and inflating the airbag, the method further includes: detecting performance of the air pump, and determining a first weight parameter based on the performance of the air pump; and the determining a wearing detection result of the wearable device based on the volume expansion rate of the airbag within the T seconds includes: determining the wearing detection result of the wearable device based on the volume expansion rate of the airbag within the T seconds and the first weight parameter.

In this embodiment of this application, the wearable device may first evaluate the performance of the air pump, and then determine, based on the performance of the air pump and the volume expansion rate of the airbag, whether the wearable device is normally worn. This ensures accuracy of a physiological parameter measurement result, without adding hardware, and without incurring additional costs.

With reference to the first aspect, in some implementations of the first aspect, before the determining that a preset condition is met, and inflating the airbag, the method further includes: determining a wearing posture of a user, and determining a second weight parameter based on the wearing posture; and the determining a wearing detection result of the wearable device based on the volume expansion rate of the airbag within the T seconds includes: determining the wearing detection result of the wearable device based on the volume expansion rate of the airbag within the T seconds, the first weight parameter, and the second weight parameter.

In this embodiment of this application, the wearable device may first detect the wearing posture of the user, and then determine, based on the wearing posture of the user and the volume expansion rate of the airbag, whether the wearable device is normally worn. This ensures accuracy of a physiological parameter measurement result, without adding hardware, and without incurring additional costs.

With reference to the first aspect, in some implementations of the first aspect, the detecting performance of the air pump includes: inflating the airbag; when a pressure of the airbag reaches a first pressure, performing deflation; and

determining the performance of the air pump based on a deflation rate.

With reference to the first aspect, in some implementations of the first aspect, the detecting performance of the air pump includes: inflating the airbag, where a duty cycle of the air pump is a first duty cycle; when a pressure of the airbag reaches a second pressure, determining an inflation time period; and determining the performance of the air pump based on the inflation time period.

With reference to the first aspect, in some implementations of the first aspect, before the determining that a preset condition is met, and inflating the airbag, the method further includes: determining a wearing posture of a user, and determining a second weight parameter based on the wearing posture; and the determining a wearing detection result of the wearable device based on the volume expansion rate of the airbag within the T seconds includes: determining the wearing detection result of the wearable device based on the volume expansion rate of the airbag within the T seconds, and the second weight parameter.

With reference to the first aspect, in some implementations of the first aspect, the detecting a volume expansion rate of the airbag within the T seconds includes: determining the volume expansion rate of the airbag within the T seconds according to the following formula:

$$\varepsilon_t = \frac{P_0}{P_t}(1 + \frac{W'(t)}{n'_0})$$

$$\varepsilon = \frac{\sum_{t=0}^{T} \varepsilon_t}{T}$$

$P_0$ is a pressure inside the airbag when the airbag is not inflated, $P_t$ is a pressure inside the airbag at a moment t when the airbag is inflated, W'(t) is a difference between an inflation rate and a deflation rate at the moment t, $n'_0$ is an amount of substance of air, and $\varepsilon$ is the volume expansion rate of the airbag within the T seconds. T is a time period for inflating the airbag, and T>0.

With reference to the first aspect, in some implementations of the first aspect, the method further includes: determining adjustment prompt information based on the volume expansion rate of the airbag within the T seconds; and displaying the adjustment prompt information in the first interface.

In this embodiment of this application, the wearable device may display the adjustment prompt information based on the volume expansion rate. The adjustment prompt information may prompt the user for an adjustment manner and an adjustment amplitude, so that the user can perform more accurate tightness adjustment. This ensures accuracy of a physiological parameter measurement result, without adding hardware, and without incurring additional costs.

With reference to the first aspect, in some implementations of the first aspect, the determining a wearing detection result of the wearable device based on the volume expansion rate of the airbag within the T seconds includes: when the volume expansion rate of the airbag within the T seconds is greater than a first threshold, determining that the wearable device is worn too loose; when the volume expansion rate of the airbag within the T seconds is less than a second threshold, determining that the wearable device is worn too tight; or when the volume expansion rate of the airbag within the T seconds is greater than or equal to the second threshold and is less than or equal to the first threshold, determining that the wearable device is normally worn.

With reference to the first aspect, in some implementations of the first aspect, the detecting that a preset condition is met includes: detecting an operation of triggering physiological parameter measurement by a user; and/or detecting an operation of triggering wearable device wearing detection by the user; and/or detecting that a difference between a measured physiological parameter value and an average value of physiological parameter values measured in a first time period is greater than a third threshold.

According to a second aspect, a wearable device is provided. The wearable device includes one or more processors, one or more memories, and a blood pressure measurement component. The one or more memories store one or more computer programs. The one or more computer programs include instructions, and when the instructions are executed by the one or more processors, the foregoing aspect or any one of the possible implementations of the foregoing aspect is performed.

According to a third aspect, a computer-readable storage medium is provided. The computer-readable storage medium includes a computer program or instructions, and when the computer program or the instructions are run on a computer, the method according to the first aspect and any one of the possible implementations of the first aspect is performed.

According to a fourth aspect, a computer program product is provided. The computer program product includes a computer program or instructions, and when the computer program or the instructions are run on a computer, the method according to the first aspect and any one of the possible implementations of the first aspect is performed.

According to a fifth aspect, a computer program is provided. When the computer program is run on a computer, the method according to the first aspect and any one of the possible implementations of the first aspect is performed.

According to a sixth aspect, an embodiment of this application provides a graphical user interface of a wearable device. The wearable device has a display, one or more memories, and one or more processors. The one or more processors are configured to execute one or more computer programs stored in the one or more memories. The graphical user interface includes a graphical user interface displayed when the wearable device performs the technical solutions according to the foregoing aspects and any possible design of the foregoing aspects.

According to a seventh aspect, an embodiment of this application provides a wearable device. The wearable device includes modules/units that perform the method according to the foregoing aspects or any one of the possible designs of the foregoing aspects. These modules/units may be implemented by hardware, or may be implemented by hardware executing corresponding software.

For beneficial effects of the second aspect to the seventh aspect, refer to the beneficial effects of the first aspect. Details are not described again.

**BRIEF DESCRIPTION OF DRAWINGS**

FIG. 1 is a diagram of a structure of a wearable device according to an embodiment of this application;
FIG. 2(a) to FIG. 2(f) show a group of GUIs according to an embodiment of this application;
FIG. 3(a) to FIG. 3(d) show a group of GUIs according to an embodiment of this application;
FIG. 4 shows a group of GUIs according to an embodiment of this application;
FIG. 5(a) to FIG. 5(d) show a group of GUIs according to an embodiment of this application;
FIG. 6 is a schematic flowchart of a wearing detection method for a wearable device according to an embodiment of this application;
FIG. 7 is a schematic flowchart of a wearing detection method for a wearable device according to an embodiment of this application;
FIG. 8 is a schematic flowchart of a wearing detection method for a wearable device according to an embodiment of this application;
FIG. 9 is a schematic flowchart of a wearing detection method for a wearable device according to an embodiment of this application;
FIG. 10 is a block diagram of a wearable device according to an embodiment of this application; and
FIG. 11 is a diagram of a structure of a wearable device according to an embodiment of this application.

**DESCRIPTION OF EMBODIMENTS**

The following describes technical solutions of embodiments in this application with reference to accompanying drawings.

Terms used in the following embodiments are merely intended to describe specific embodiments, but are not intended to limit this application. The terms "one", "a" and "this" of singular forms used in this specification and the appended claims of this application are also intended to include expressions such as "one or more", unless otherwise specified in the context clearly. It should be further understood that in the following embodiments of this application, "at least one" and "one or more" means one, two, or more. The term "and/or" is used to describe an association relationship between associated objects and represents that three relationships may exist. For example, A and/or B may represent the following cases: Only A exists, both A and B exist, and only B exists, where A and B may be singular or plural. The character "/" generally indicates an "or" relationship between the associated objects.

Reference to "an embodiment", "some embodiments", or the like described in this specification indicates that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

A wearable device provided in this application may be a portable device that can be integrated into clothes or an accessory of a user, has a computing function, and can be connected to a mobile phone and various terminal devices. For example, the wearable device 100 may be a smartwatch, a blood pressure band, an upper arm blood pressure meter, or the like. A type of the wearable device is not specifically limited in this application.

It should be noted that the wearable device provided in embodiments of this application has a blood pressure measurement function and/or a blood oxygen measurement function and/or a heart rate measurement function.

The wearable device usually performs blood pressure measurement by using a principle of an oscillometric method. Specifically, a waveform trace from a blood vessel wall is obtained by using a vibration caused by collision of blood with the blood vessel wall during blood flow, and a blood pressure value is obtained by using a relationship between the waveform trace and an arterial blood pressure. During specific measurement, the wearable device needs to be worn on a limb of a person, and an airbag of the wearable device is used to compress an artery of a measured user. When a blood pressure in the artery of the measured user impacts the airbag, pressure fluctuation occurs in an air pressure inside the airbag. Then, a blood pressure indicator of the measured user is calculated based on a fluctuation status of the air pressure inside the airbag.

The wearable device usually uses a photoelectric measurement method for heart rate measurement. Specifically, a light beam is emitted to a skin surface, light intensity at different time is obtained by using a blood flow change caused by a heartbeat, and a heart rate value is obtained by using the light intensity at different time. Similar to blood pressure measurement, heart rate measurement requires the wearable device to be worn on a limb of the person. When a light beam with a fixed wavelength is irradiated on a skin surface, the light beam transmits light to a sensor in a transmission or reflection manner. In this process, attenuation occurs due to absorption of skin, muscle and blood, resulting in a decrease in light intensity detected by the sensor. Absorption of light by the skin, muscle tissue, and the like is constant in entire blood circulation, but a blood volume in the skin shows a pulsating change under an action of the heart. When the heart contracts, a peripheral blood volume is the largest, light absorption is the largest, and detected light intensity is the smallest. However, when the heart expands, on the contrary, detected light intensity is the largest, that is, the detected light intensity shows a pulsatile change, so that a heart rate value of the user is obtained through calculation.

The wearable device usually uses a photoelectric measurement method for blood oxygen measurement. Specifically, red light and infrared light are emitted to a skin surface. Because oxygen-saturated hemoglobin is reddish, when the red light is received, a reflectivity of the red light is higher, and a reflectivity of the infrared light is lower. On the contrary, when oxygen-saturated hemoglobin contains insufficient oxygen, a reflectivity of red light is lower, and a reflectivity of infrared light is higher. A blood oxygen value is obtained by analyzing different reflectivities of the red light and the infrared light. Blood oxygen measurement is similar to heart rate measurement. The wearable device needs to be worn on a limb of the person.

In conclusion, when the wearable device performs the foregoing measurement functions, the wearable device needs to be worn on a limb of the person. However, a wearing tightness degree affects accuracy of a measurement result. Blood pressure measurement is used as an example for description. For example, the wearable device is worn too loose, and the airbag needs to be inflated more to press the artery of the measured user. In this case, the pressure inside the airbag is higher compared with that in normal wearing, resulting a higher measured blood pressure value. On the contrary, when the wearable device is worn too tight, the pressure inside the airbag is lower compared with that in normal wearing, resulting a lower measured blood pressure value. It can be learned that the accuracy of the measurement result is affected if the wearable device is worn loose. Based on this, embodiments of this application provide a wearing detection method for a wearable device and a wearable device.

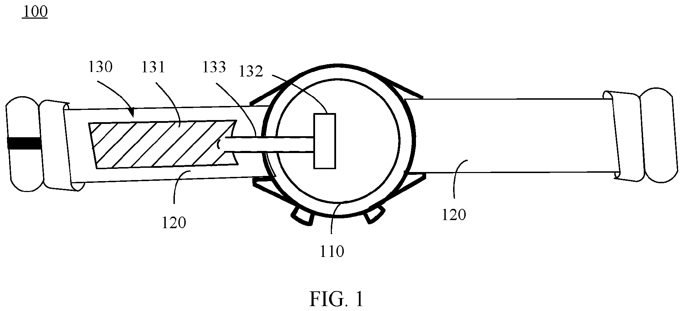

FIG. 1 is a diagram of a structure of a wearable device 100 according to an embodiment of this application. In some embodiments, the wearable device 100 may be a smartwatch or a band that can be worn around a wrist of a user.

As shown in FIG. 1, the wearable device 100 may include a main body 110, a wrist strap 120, and a blood pressure detection component 130. The wrist strap 120 may enclose and be attached to a body part of the user, for example, the wrist, an upper arm, a foot wrist, or another part of the body of the user, to attach the main body 110 to a to-be-detected part of the user. The wearable device 100 may implement a blood pressure measurement function by using the blood pressure detection component 130.

The blood pressure detection component 130 may include an airbag 131, an air pump 132, and a barometric pressure sensor (not shown in the figure). The airbag 131 may be disposed on an inner surface of the wrist strap 120. The air pump 132 may be disposed inside the main body 110, communicated with the airbag 131 through an air tube 133, and configured to inflate or deflate the airbag 131. The barometric pressure sensor may also be disposed inside the main body 110, connected to the airbag 131, and configured to detect an air pressure change of the airbag 131.

It may be understood that the inner surface of the wrist strap 120 may be a surface that is of the wrist strap 20 and that is in contact with the body part of the user.

For example, when the user performs blood pressure measurement by using the wearable device 100 worn on the wrist, the wearable device 100 may control the air pump 132 to start an inflating action, and the air pump 132 may inflate the airbag 31 by using the air tube 133, to pressurize and expand the airbag 131, pressing a radial artery of the wrist. In this case, an air pressure inside the airbag 131 may generate pressure fluctuation. The wearable device 100 may obtain an air pressure wave signal in the airbag 131 by using the barometric pressure sensor, and calculate a diastolic pressure and a systolic pressure of the user based on the air pressure wave signal, to implement the blood pressure measurement function.

After a blood pressure measurement process ends, the wearable device 100 may control the air pump 132 to stop the inflating action, and air in the airbag 131 may be discharged by using the air pump 132, to maintain a state in which a blood pressure can be remeasured.

Optionally, in some embodiments, the wearable device may further include a photoplethysmography (photoplethysmography, PPG) sensor. The PPG sensor is configured to collect a heart rate and blood oxygen of the user.

The following first describes, with reference to a graphical user interface (graphical user interface, GUI), several scenarios to which a wearing detection method for a wearable device provided in an embodiment of this application is applicable.

It should be noted that in the following GUI, an example in which the wearable device is a smartwatch is used for description, but this should not be understood as a specific limitation on this embodiment of this application.

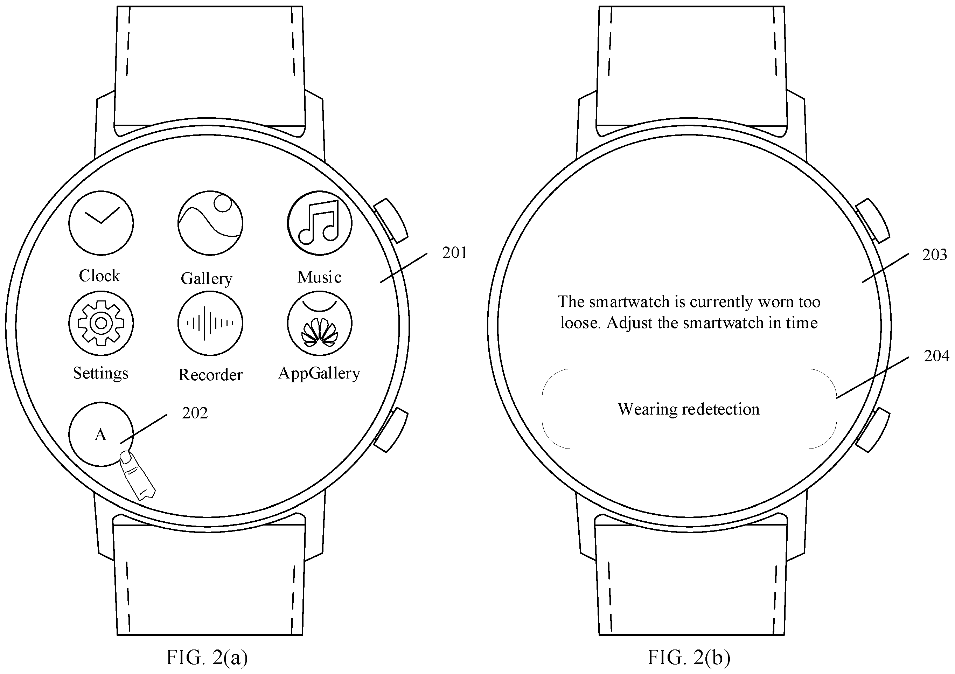

FIG. 2(a) to FIG. 2(f) show a group of GUIs according to an embodiment of this application.

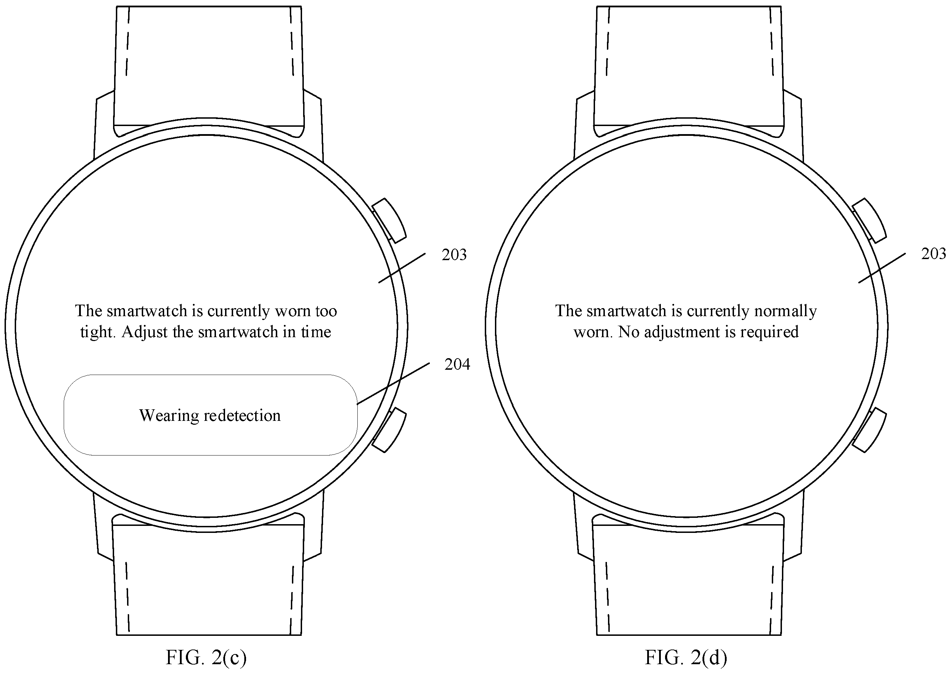

As shown in FIG. 2(a), a smartwatch displays an interface 201, and the interface 201 is a home screen. An icon 202 is displayed in the interface 201 of the smartwatch, and the icon 202 corresponds to a wearing detection function. When detecting an operation of tapping the icon 202 by a user, the smartwatch may detect a wearing tightness degree of the smartwatch in response to the operation. When it is determined that the smartwatch is worn too loose, a GUI shown in FIG. 2(b) may be displayed. When it is determined that the smartwatch is worn too tight, a GUI shown in FIG. 2(c) may be displayed. When the smartwatch is normally worn, a GUI shown in FIG. 2(d) may be displayed.

As shown in FIG. 2(b), when the smartwatch completes detection and determines that the smartwatch is worn too loose, the smartwatch may display a detection result in an interface 203: "The smartwatch is currently worn too loose. Adjust the smartwatch in time".

As shown in FIG. 2(c), when the smartwatch completes detection and determines that the smartwatch is worn too tight, the smartwatch may display a detection result in the interface 203: "The smartwatch is currently worn too tight. Adjust the smartwatch in time".

As shown in FIG. 2(d), when the smartwatch completes detection and determines that the smartwatch is normally worn, the smartwatch may display a detection result in the interface 203: "The smartwatch is currently normally worn. No adjustment is required".

Optionally, in some embodiments, the smartwatch may further display a wearing redetection control 204 in the interface 203. The smartwatch detects an operation of tapping the wearing redetection control 204 by the user, and in response to the operation, may redetect the wearing tightness degree of the smartwatch, and redisplay a detection result in the interface 203.

For example, the smartwatch detects the wearing tightness degree for the first time, detects that the smartwatch is worn too loose, and may output a detection result of wearing too loose. After viewing the detection result, a user may perform corresponding adjustment. After adjustment, the user does not need to return from the interface 203 to the interface 201 to tap the icon 202 in the interface 201, but may directly tap the wearing redetection control 204 in the interface 203 to trigger the wearing detection function of the smartwatch.

It should be noted that for a method for detecting wearing tightness of the smartwatch, refer to the following descriptions. Details are not described herein.

In this embodiment of this application, the smartwatch may detect whether the user wears the smartwatch normally, to further ensure accuracy of performing functions such as blood pressure measurement, heart rate measurement, and blood oxygen measurement. This helps improve user experience.

Optionally, in some embodiments, when determining that the smartwatch is worn too tight or too loose, the smartwatch may further display adjustment prompt information.

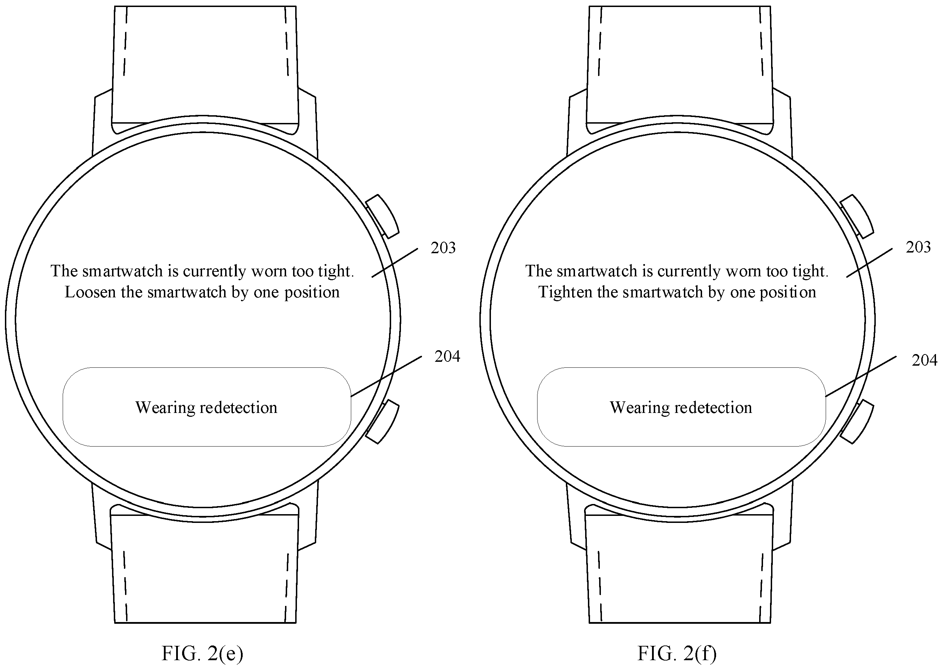

For example, as shown in FIG. 2(e), when determining that the smartwatch is worn too tight, the smartwatch may display adjustment prompt information: "Loosen the watch strap by one position".

For example, as shown in FIG. 2(f), when determining that the smartwatch is worn too loose, the smartwatch may display adjustment prompt information: "Tighten the watch strap by one position".

It may be understood that FIG. 2(e) and FIG. 2(f) are merely examples, and the adjustment prompt information displayed by the smartwatch is determined based on the wearing tightness degree of the user, and is not fixed.

In this embodiment of this application, the smartwatch may detect whether the user wears the smartwatch normally. When determining that the user wears the smartwatch too tight or too loose, the smartwatch may output the adjustment prompt information, to prompt the user to perform adjustment. This further ensures accuracy of performing functions such as blood pressure measurement, heart rate measurement, and blood oxygen measurement, and helps improve user experience.

It should be noted that in the example shown in FIG. 2(a), the home screen 201 includes the control used to trigger the wearing detection function. However, this is not limited in this embodiment of this application. In some other embodiments of this application, an interface and a service widget of an application may include a control used to trigger the wearing detection function.

It should be further noted that, in this embodiment of this application, an operation of triggering wearing detection includes tapping the control used to trigger the wearing detection function by the user, pressing a crown (for example, pressing the crown three consecutive times) by the user, rotating the crown (for example, rotating the crown for two turns) by the user, a preset gesture, and the like.

FIG. 2(a) to FIG. 2(f) describe GUIs in which the smartwatch performs wearing detection in response to the operation of triggering the wearing detection function by the user. The following describes GUIs in which the smartwatch performs wearing detection during physiological parameter measurement (for example, blood pressure measurement, or heart rate measurement). The following mainly uses blood pressure measurement as an example for description.

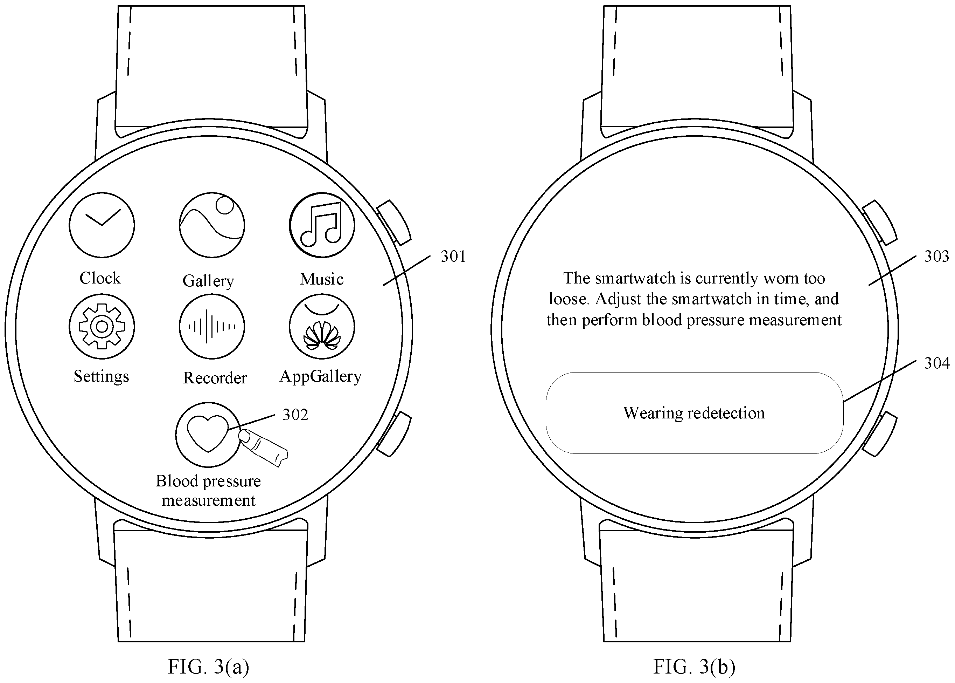

FIG. 3(a) to FIG. 3(d) show another group of GUIs according to an embodiment of this application.

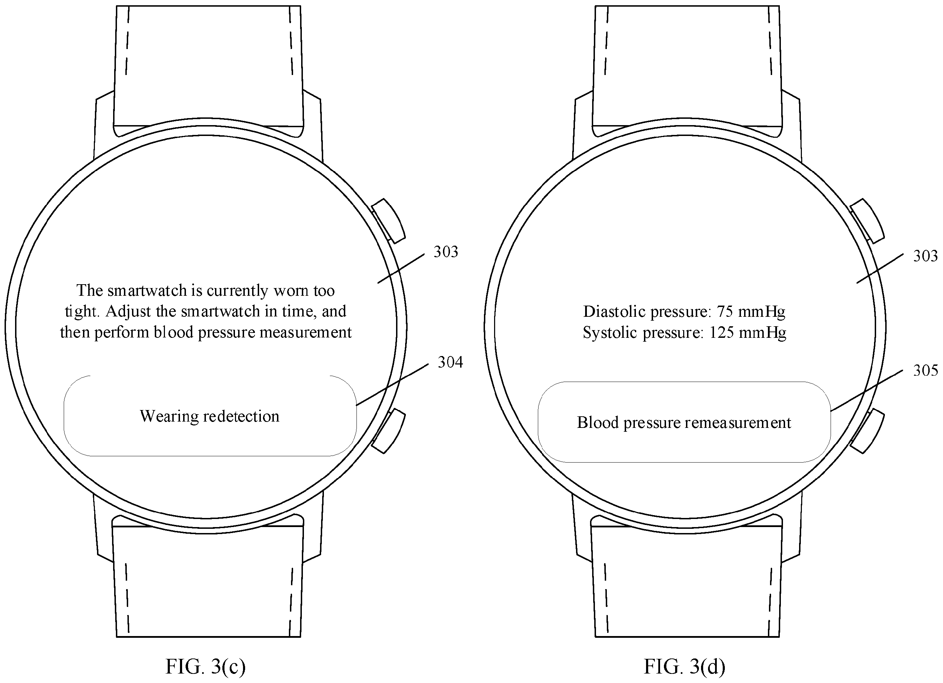

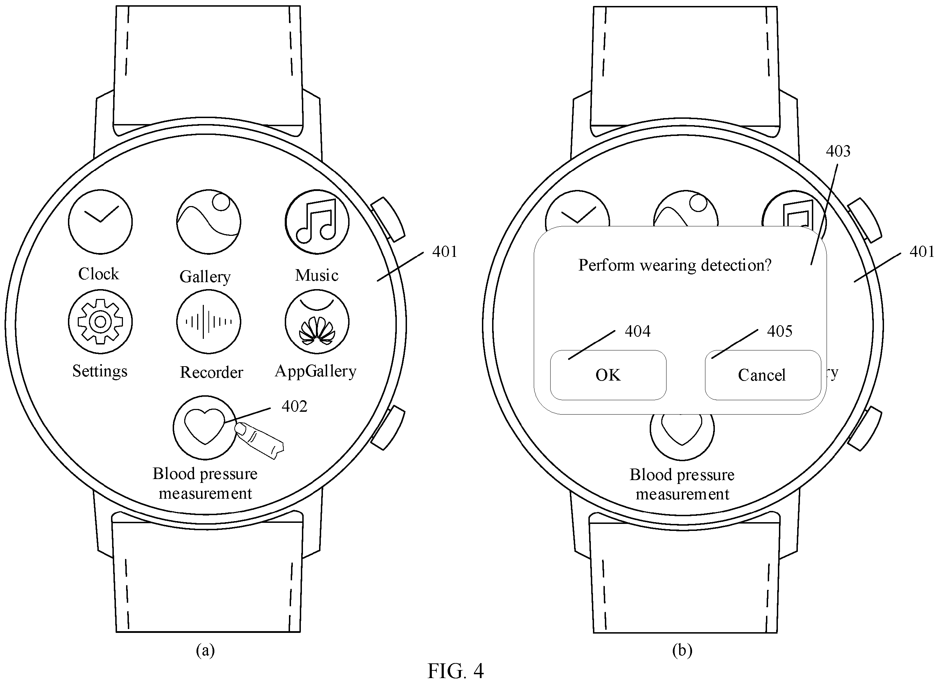

As shown in FIG. 3(a) to FIG. 3(d), a smartwatch displays an interface 301, and the interface 301 is a home screen. An icon 302 is displayed in the interface 301 of the smartwatch, and the icon 302 corresponds to a blood pressure measurement function. When detecting an operation of tapping the icon 302 by a user, the smartwatch may perform blood pressure measurement in response to the operation. Before performing blood pressure measurement, the smartwatch may first perform wearing detection. The smartwatch may automatically trigger wearing detection, as shown as the GUIs in FIG. 3(a) to FIG. 3(d), or may trigger wearing detection in response to an operation manually determined by a user, as shown as GUIs in FIG. 4. When it is determined that the smartwatch is worn too loose, a GUI shown in FIG. 3(b) may be displayed. When it is determined that the smartwatch is worn too tight, a GUI shown in FIG. 3(c) may be displayed. When the smartwatch is normally worn, blood pressure measurement may be performed, and a GUI shown in FIG. 3(d) may be displayed.

As shown in FIG. 3(b), when determining that the smartwatch is worn too loose, the smartwatch may display a detection result in an interface 303: "The smartwatch is currently worn too loose. Adjust the smartwatch in time, and then perform blood pressure measurement".

As shown in FIG. 3(c), when determining that the smartwatch is worn too tight, the smartwatch may display a detection result in the interface 303: "The smartwatch is currently worn too tight. Adjust the smartwatch in time, and then perform blood pressure measurement".

As shown in FIG. 3(d), when determining that the smartwatch is normally worn, the smartwatch may perform blood pressure measurement and display a blood pressure measurement result in the interface 203.

In this embodiment of this application, before performing blood pressure measurement, the smartwatch may detect whether the user wears the smartwatch normally, to further ensure accuracy of a blood pressure measurement function. This helps improve user experience.

Optionally, in some embodiments, when determining that the smartwatch is worn too loose or too tight, the smartwatch may further display a wearing redetection control 304 in the interface 303. The smartwatch detects an operation of tapping the wearing redetection control 304 by the user, and may perform wearing redetection in response to the operation.

Optionally, in some embodiments, when the smartwatch determines that the smartwatch is normally worn and completes blood pressure measurement, the smartwatch may further display a blood pressure remeasurement control 305 in the interface 303. The smartwatch detects an operation of tapping the blood pressure remeasurement control 305 by the user, and may perform blood pressure remeasurement in response to the operation.

Optionally, in some embodiments, when determining that the smartwatch is worn too tight or too loose, the smartwatch may further display adjustment prompt information. It should be understood that, for descriptions of displaying the adjustment prompt information by the smartwatch, refer to the descriptions of FIG. 2(e) and FIG. 2(f). For brevity, details are not described herein again.

In this embodiment of this application, before performing blood pressure measurement, the smartwatch may detect whether the user wears the smartwatch normally. When determining that the user wears the smartwatch too tight or too loose, the smartwatch may output the adjustment prompt information, to prompt the user to perform adjustment. This further ensures accuracy of blood pressure measurement, and helps improve user experience.

In the GUIs shown in FIG. 3(a) to FIG. 3(d), the smartwatch may automatically detect wearing tightness before performing blood pressure measurement. In some other embodiments of this application, the smartwatch may determine, based on a user operation, whether to detect wearing tightness before performing blood pressure measurement. The following provides descriptions with reference to FIG. 4.

FIG. 4 shows another group of GUIs according to an embodiment of this application.

As shown in (a) in FIG. 4, a smartwatch displays an interface 401, and the interface 401 may be a home screen. An icon 402 is displayed in the interface 401 of the smartwatch, and the icon 402 corresponds to a blood pressure measurement function. When detecting an operation of tapping the icon 402 by a user, the smartwatch may display a GUI shown in (b) in FIG. 4 in response to the operation.

As shown in (b) in FIG. 4, the smartwatch may display an option box 403 in response to an operation of tapping the icon 402 by the user. The option box 403 is used to output prompt information, where the prompt information is used to prompt the user whether to perform wearing detection. When detecting an operation of tapping an OK control 404 by the user, the smartwatch may first perform wearing detection. Then, when determining that the smartwatch is normally worn, the smartwatch performs blood pressure measurement. When the smartwatch detects an operation of tapping a cancel control 405 by the user, the smartwatch may perform blood pressure measurement.

It may be understood that, when the smartwatch performs wearing tightness in response to the operation of tapping the OK control 404 by the user, if it is detected that the smartwatch is worn too tight or too loose, similar to the GUIs shown in FIG. 2(a) to FIG. 2(f) or FIG. 3(a) to FIG. 3(d), the smartwatch may display a detection result. For specific descriptions, refer to the foregoing description. Details are not described herein again.

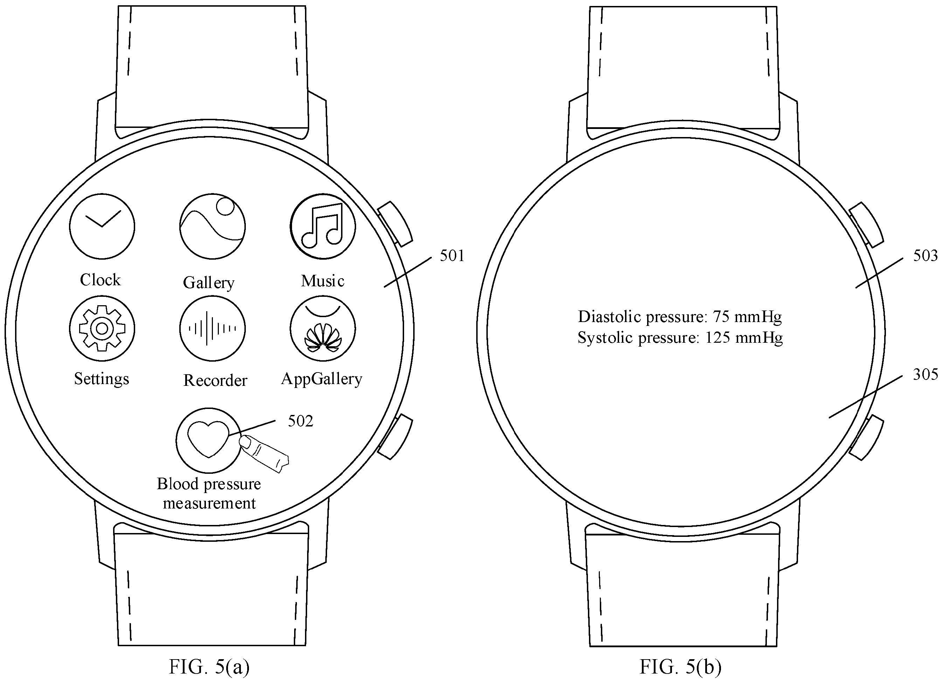

In the GUIs shown in FIG. 3(a) to FIG. 3(d) and FIG. 4, wearing detection is performed before blood pressure measurement. In some other embodiments of this application, the smartwatch may further perform wearing detection after completing blood pressure measurement. The following provides descriptions with reference to FIG. 5(a) to FIG. 5(d).

FIG. 5(a) to FIG. 5(d) show another group of GUIs according to an embodiment of this application.

As shown in FIG. 5(a), a smartwatch displays an interface 501, and the interface 501 may be a home screen. An icon 502 is displayed in the interface 501 of the smartwatch, and the icon 502 corresponds to a blood pressure measurement function. When detecting an operation of tapping the icon 502 by a user, the smartwatch may display a GUI shown in FIG. 5(b) in response to the operation.

As shown in FIG. 5(b), the smartwatch may perform blood pressure measurement in response to the operation of tapping the icon 502 by the user, and display a blood pressure measurement result in an interface 503.

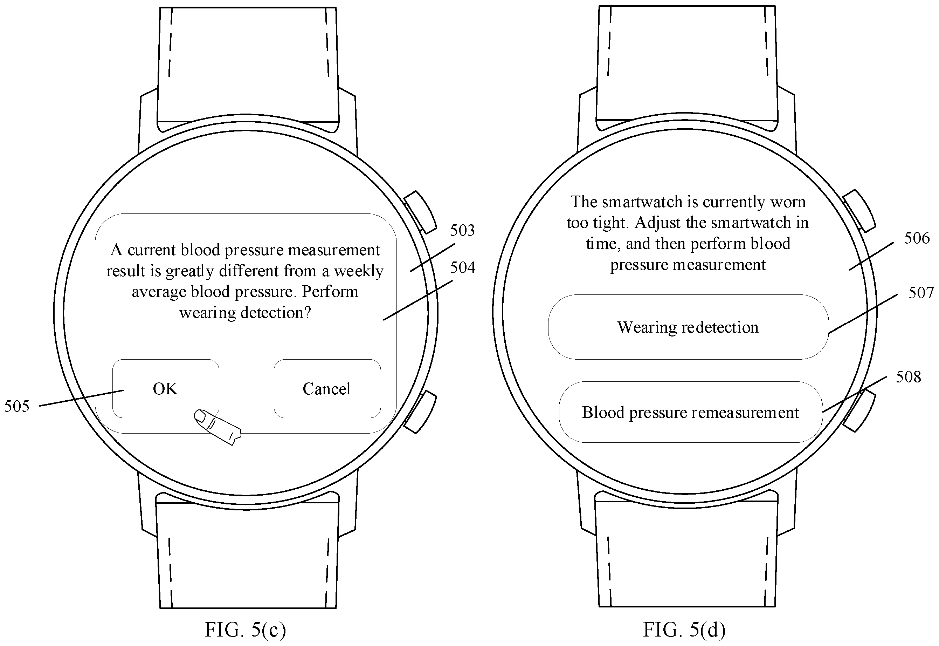

As shown in FIG. 5(c) and FIG. 5(d), the smartwatch may store an average value of blood pressure measurement results in a past time period (for example, one week). The smartwatch may compare a difference between the average value and the current blood pressure measurement result. When the difference is greater than a threshold, the smartwatch may display an option box 504. The option box 504 is used to output prompt information, where the prompt information is used to prompt a user whether to perform wearing detection. When detecting an operation of tapping an OK control 505 by the user, the smartwatch may perform wearing detection. After detection is completed, the smartwatch displays a detection result in an interface 506: "The smartwatch is currently worn too loose. Adjust the smartwatch in time, and then perform blood pressure measurement".

Optionally, in some embodiments, the smartwatch may further display adjustment prompt information in the interface 506. For specific descriptions, refer to the foregoing description. Details are not described herein again.

Optionally, in some embodiments, the smartwatch may further display a wearing redetection control 507 and/or a blood pressure remeasurement control 508 in the interface 506. For specific descriptions, refer to the foregoing description. Details are not described herein again.

In this embodiment of this application, after completing blood pressure measurement, the smartwatch may compare a current blood pressure measurement result with a previous blood pressure measurement result. If a difference is relatively large, the smartwatch may perform wearing detection to prompt the user to perform adjustment. This helps improve user experience.

With reference to the foregoing several GUI scenarios to which the wearing detection method for a wearable device provided in an embodiment of this application is applicable, the following describes a wearing detection method for a wearable device provided in an embodiment of this application.

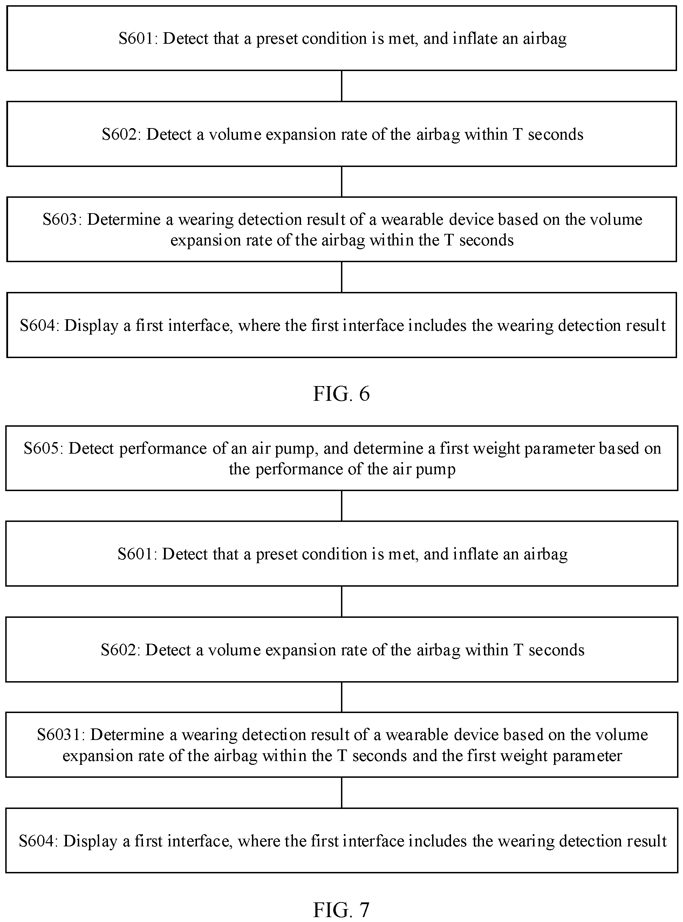

FIG. 6 is a schematic flowchart of a wearing detection method 600 for a wearable device according to an embodiment of this application. As shown in FIG. 6, the method includes the following steps.

S601: Detect that a preset condition is met, and inflate an airbag.

Specifically, when detecting that the preset condition is met, a wearable device may inflate the airbag, where an inflation time period is T seconds, and T>0.

In some embodiments, the detecting that the preset condition is met is: detecting an operation of triggering wearing detection by a user.

For example, as shown in FIG. 2(a), the smartwatch detects the operation of tapping the icon 202 by the user, where the icon 202 corresponds to the wearing detection function.

For another example, as shown in FIG. 2(b) to FIG. 2(f), the smartwatch detects the operation of tapping the wearing redetection control 204 by the user

For another example, as shown in FIG. 3(b) to FIG. 3(d), the smartwatch detects the operation of tapping the wearing redetection control 304 by the user.

For another example, as shown in FIG. 4, the option box 403 includes the OK control 404 and the cancel control 405. The option box 403 is used to output prompt information, where the prompt information is used to prompt the user whether to perform wearing detection, and the smartwatch detects the operation of tapping the OK control 404 by the user.

For another example, the smartwatch detects an operation of pressing a crown three consecutive times by the user.

For another example, the smartwatch detects an operation of rotating the crown for two turns by the user.

For another example, the smartwatch detects a preset gesture of the user, where the preset gesture corresponds to the wearing detection function.

In some embodiments, the detecting that the preset condition is met is: detecting an operation of triggering physiological parameter measurement by the user. The physiological parameter includes one or more of the following: a blood pressure, a heart rate, and blood oxygen.

For example, as shown in FIG. 3(a), the smartwatch detects the operation of tapping the icon 302 by the user, where the icon 302 corresponds to the blood pressure measurement function.

In some embodiments, the detecting that the preset condition is met is: detecting that a difference between a measured physiological parameter value and an average value of physiological parameter values measured in a first time period is greater than a threshold.

For example, as shown in FIG. 5(c), when the smartwatch detects that a difference between an average value of blood pressure values measured in previous one week and the current blood pressure measurement result is greater than the threshold, wearing detection may be performed.

S602: Detect a volume expansion rate of the airbag within the T seconds.

Specifically, when the airbag is inflated by using an air pump, the wearable device may detect the volume expansion rate of the airbag to determine wearing tightness. The volume expansion rate may be understood as a parameter representing an airbag expansion speed. When the airbag expands quickly, the volume expansion rate of the airbag is large. When the airbag expands slowly, the volume expansion rate of the airbag is small. When the airbag does not press a skin of the user, the volume expansion rate of the airbag may remain unchanged. When the airbag starts to press the skin of the user, the volume expansion rate of the airbag starts to slow down due to impact of resistance from the skin of the user. It is not difficult to understand that when the airbag is inflated at a same inflation rate, wearing the wearable device tighter indicates that the airbag encounters resistance from the skin of the user earlier, and the volume expansion rate of the airbag is smaller. Therefore, a wearing tightness degree of the wearable device may be determined by detecting the volume expansion rate of the airbag.

In some embodiments, due to a duty cycle of an air pump during operation, the airbag is deflated in an operation interval of the air pump. In addition, as a volume of the airbag increases, the resistance from the skin to the airbag also increases. Therefore, a deflation rate of the airbag in the operation interval of the air pump also increases. It is not difficult to understand that, at same time, wearing the wearable device tighter indicates that the airbag encounters larger resistance from the skin, and the deflation rate of the airbag in the operation interval of the air pump is larger. Therefore, the wearable device may determine the volume expansion rate of the airbag within the T seconds according to the formulas (1) and (2).

$$\varepsilon_t = \frac{P_0}{P_t}\left(1 + \frac{W'(t)}{n'_0}\right) \tag{1}$$

$$\varepsilon = \frac{\sum_{t=0}^{T} \varepsilon_t}{T} \tag{2}$$

$P_0$ is a pressure inside the airbag when the airbag is not inflated, $P_t$ is a pressure inside the airbag at a moment t when the airbag is inflated, $W'(t)$ is a difference between an inflation rate and a deflation rate at the moment t, $n'_0$ is an amount of substance of air, and $\varepsilon$ is the volume expansion rate of the airbag within the T seconds. T is a time period for inflating the airbag, and T>0.

$W'(t)$ is determined based on an inflation rate and a deflation rate at the moment t. The inflation rate and the deflation rate are quantities related to the air pump and the pressure of the airbag. When a hardware specification of the air pump is fixed, the inflation rate and the deflation rate may be determined based on the duty cycle of the air pump and the pressure of the airbag.

For example, an air inflation rate and a deflation rate of a same air pump under different duty cycles and different pressures of the airbag may be obtained through experimental measurement, and then the wearable device may store this measurement result, so that the measurement result may be used for calculation of the volume expansion rate of the airbag.

It should be noted that the foregoing formulas (1) and (2) are merely examples, and are not construed as a limitation on this application. The wearing detection method for a wearable device provided in this embodiment of this application, in which the volume expansion rate of the airbag is measured by using another method, should also fall within the protection scope of this application.

S603: Determine a wearing detection result of the wearable device based on the volume expansion rate of the airbag within the T seconds.

Specifically, after determining the volume expansion rate of the airbag, the wearable device may determine the wearing detection result based on a relationship between the measured volume expansion rate and a first threshold.

In some embodiments, when the volume expansion rate is greater than the first threshold, it is determined that the device is worn too loose.

For example, if the first threshold is 2, and the measured volume expansion rate is 5, it may be determined that the wearable device is worn too loose.

In some embodiments, when the volume expansion rate is less than a second threshold, it is determined that the device is worn too tight.

For example, if the second threshold is 1.5, and the measured volume expansion rate is 1.3, it may be determined that the wearable device is worn too tight.

In some embodiments, when the volume expansion speed is greater than or equal to the second threshold and less than or equal to the first threshold, it is determined that the device is normally worn.

For example, if the first threshold is 2, the second threshold is 1.5, and the measured volume expansion rate is 1.8, it may be determined that the wearable device is normally worn.

S604: Display a first interface, where the first interface includes the wearing detection result.

Specifically, after determining the wearing detection result, the wearable device may display the wearing detection result in the first interface.

For example, as shown in FIG. 2(b), the smartwatch displays the wearing detection result in the interface 203.

In this embodiment of this application, the wearable device may determine, by detecting the volume expansion rate of the airbag, whether the wearable device is normally worn. This ensures accuracy of a physiological parameter measurement result, without adding hardware, and without incurring additional costs.

Because the air pump is affected by a manufacturing deviation, air path blockage during use, and the like, a large deviation may exist when the air pump inflates the airbag, resulting in an inaccurate wearing detection result.

For example, it is assumed that a user #1 normally wears a wearable device #1, a user #2 normally wears a wearable device #2, the wearable device #1 is equipped with an air pump #1, and the wearable device #2 is equipped with an air pump #2. The air pump #1 and the air pump #2 are air pumps of a same model. Air path blockage occurs in the air pump #1 and does not occur in the air pump #2. The air pump #1 and the air pump #2 respectively inflate the airbag #1 and the airbag #2 at same power. It is not difficult to understand that, due to air path blockage of the air pump #1, a volume expansion rate of the airbag #1 is less than a volume expansion rate of the airbag #2, and may be less than the second threshold. Therefore, it is determined that the user #1 wears the wearable device #1 too tight.

In conclusion, as shown in FIG. 7, in some embodiments of this application, before S601 is performed, the method further includes the following steps.

S605: Detect performance of the air pump, and determine a first weight parameter based on the performance of the air pump.

Specifically, the wearable device may detect the performance of the air pump, and determine the first weight parameter based on the performance of the air pump.

The determining a wearing detection result of the wearable device based on the volume expansion rate of the airbag within the T seconds in S603 includes:

S6031: Determine the wearing detection result of the wearable device based on the volume expansion rate of the airbag within the T seconds and the first weight parameter.

Specifically, the wearable device may determine a first weighted volume expansion rate of the airbag within the T seconds based on the volume expansion rate of the airbag within the T seconds and the first weight parameter, and then determine the wearing detection result of the wearable device based on the first weighted volume expansion rate of the airbag within the T seconds.

In some embodiments, the wearable device may determine the first weighted volume expansion rate of the airbag within the T seconds according to formulas (3) and (4).

$$\beta_t = \frac{P_0}{P_t}(1 + \frac{W'(t)}{n'_0}) \times A \tag{3}$$

$$\beta = \frac{\sum_{t=0}^{T} \beta_t}{T} \tag{4}$$

$\beta_t$ is a first weighted volume expansion rate of the airbag at the moment t, $\beta$ is the first weighted volume expansion rate of the airbag within the T seconds, and A is the first weight parameter.

It may be understood that, when the performance of the air pump is relatively poor, the air pump corresponds to a larger first weight parameter, and when the performance of the air pump is relatively good, the air pump corresponds to a smaller first weight parameter, to eliminate inaccuracy of the wearing detection result caused by performance differences between air pumps.

A method for detecting the performance of the air pump is not limited in this embodiment of this application. The following describes several possible methods for detecting the performance of the air pump.

In a possible method, the airbag is inflated; when a pressure of the airbag reaches a first pressure, deflation is performed; and the performance of the air pump is determined based on a deflation rate.

For example, the deflation rate may be determined according to formulas (5) and (6).

$$v = \frac{V_m - V_0}{m} \tag{5}$$

$$P_m V_m = P_0 V_0 \tag{6}$$

v is the deflation rate, $V_m$ is a volume of the airbag when the pressure of the airbag is the first pressure, $P_0$ is a pressure inside the airbag when the airbag is not inflated, $V_0$ is a volume of the airbag when the airbag is not inflated, $P_m$ is the first pressure, and m is time at which the pressure of the airbag changes from $P_m$ to $P_0$. It may be understood that, because $P_0$ and $V_0$ are known, and $V_m$ may be determined by using Formula (5), the deflation rate may be calculated by detecting the time at which the pressure of the airbag changes from $P_m$, to $P_0$.

In a possible method, the air pump inflates the airbag at a first duty cycle, an inflation time period is determined when a pressure of the airbag reaches a second pressure, and then the performance of the air pump is determined based on the inflation time period, where the first duty cycle is greater than 0 and less than or equal to 100%.

It may be understood that, when the inflation time period is shorter, the performance of the air pump is better, and when the inflation time period is longer, the performance of the air pump is poorer.

In this embodiment of this application, the wearable device may first evaluate the performance of the air pump, and then determine, based on the performance of the air pump and the volume expansion rate of the airbag, whether the wearable device is normally worn. This ensures accuracy of a physiological parameter measurement result, without adding hardware, and without incurring additional costs.

In addition to the impact of the performance of the air pump on the wearing detection result, a wearing posture of the user also affects the wearing detection result.

For example, the wearable device is the smartwatch. It is assumed that the smartwatch is worn too loose, and the smartwatch needs to perform wearing detection before blood pressure measurement. When the smartwatch performs blood pressure measurement, the user needs to press a wrist on the chest. This operation causes the airbag to be pressed, a measured volume expansion rate of the airbag decreases, and wearing may be incorrectly determined as normal wearing.

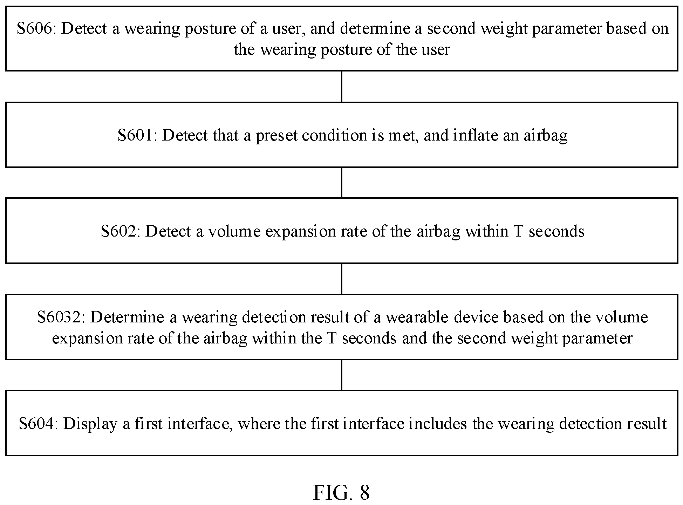

In conclusion, as shown in FIG. 8, in some embodiments of this application, before S601 is performed, the method further includes the following steps.

S606: Detect the wearing posture of the user, and determine a second weight parameter based on the wearing posture of the user.

Specifically, the wearable device may detect the wearing posture of the user, and determine the second weight parameter based on the wearing posture.

For example, the wearable device is the smartwatch. If the smartwatch detects that the user presses the wrist on the chest, the second weight parameter is 1.5. If the smartwatch does not detect that the user presses the wrist on the chest, the second weight parameter is 1.

For example, the wearable device may perform detection by using an inertia measurement unit (inertial measurement unit, IMU) or a microphone. When the wearable device detects, by using the IMU, that the wrist of the user fluctuates, and detects a heartbeat signal by using the microphone, the wearable device may determine that the user presses the wrist on the chest.

The determining a wearing detection result of the wearable device based on the volume expansion rate of the airbag within the T seconds in S603 includes:

S6032: Determine the wearing detection result of the wearable device based on the volume expansion rate of the airbag within the T seconds and the second weight parameter.

Specifically, the wearable device may determine a second weighted volume expansion rate of the airbag within the T seconds based on the volume expansion rate of the airbag within the T seconds and the second weight parameter, and then determine the wearing detection result of the wearable device based on the second weighted volume expansion rate of the airbag within the T seconds.

In some embodiments, the wearable device may determine the second weighted volume expansion rate of the airbag within the T seconds according to formulas (7) and (8).

$$\omega_t = \frac{P_0}{P_t}(1 + \frac{W'(t)}{n'_0}) \times B \tag{7}$$

$$\omega = \frac{\sum_{t=0}^{T} \omega_t}{T} \tag{8}$$

$\omega_t$ is a second weighted volume expansion rate of the airbag at the moment t, $\omega$ is the second weighted volume expansion rate of the airbag within the T seconds, and B is the second weight parameter.

In this embodiment of this application, the wearable device may first detect the wearing posture of the user, and then determine, based on the wearing posture of the user and the volume expansion rate of the airbag, whether the wearable device is normally worn. This ensures accuracy of a physiological parameter measurement result, without adding hardware, and without incurring additional costs.

Because both the performance of the air pump and the wearing posture of the user affect the wearing detection result, in some embodiments of this application, the wearable device may first detect the wearing posture of the user and the performance of the air pump.

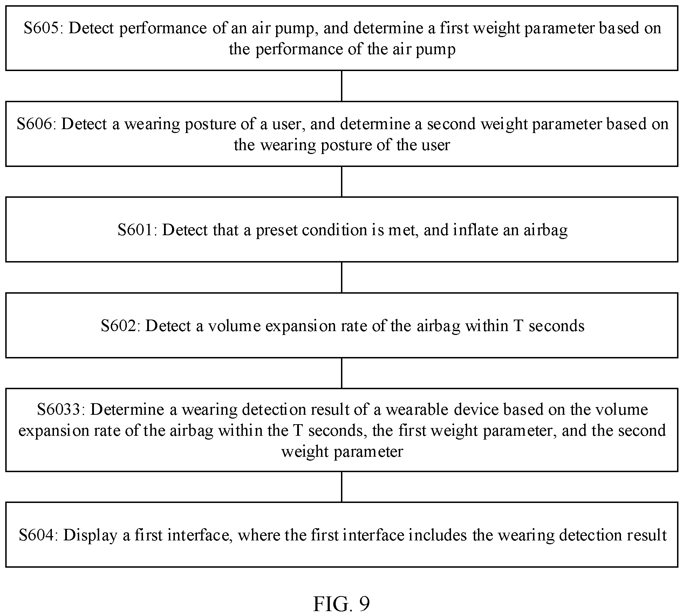

As shown in FIG. 9, before S601 is performed, the method further includes the following steps.

S605: Detect the performance of the air pump, and determine the first weight parameter based on the performance of the air pump.

S606: Detect the wearing posture of the user, and determine a second weight parameter based on the wearing posture of the user.

It should be understood that a sequence of performing S605 and S606 is not limited in this embodiment of this application. To be specific, S605 and S606 may be simultaneously performed, or S605 may be performed before S606, or S606 may be performed before S605.

The determining a wearing detection result of the wearable device based on the volume expansion rate of the airbag within the T seconds in S603 includes:

S6033: Determine the wearing detection result of the wearable device based on the volume expansion rate of the airbag within the T seconds, the first weight parameter, and the second weight parameter.

Specifically, the wearable device may determine a third weighted volume expansion rate based on the volume expansion rate of the airbag within the T seconds, the first weight parameter, and the second weight parameter, and then determine the wearing detection result of the wearable device based on the third weighted volume expansion rate of the airbag within the T seconds.

In some embodiments, the wearable device may determine the third weighted volume expansion rate of the airbag within the T seconds according to formulas (9) and (10).

$$\delta_t = \frac{P_0}{P_t}(1 + \frac{W'(t)}{n'_0}) \times A \times B \tag{9}$$

$$\delta = \frac{\sum_{t=0}^{T} \delta_t}{T} \tag{10}$$

$\delta_t$ is a third weighted volume expansion rate of the airbag at the moment t, and $\delta$ is the third weighted volume expansion rate of the airbag within the T seconds.

Optionally, in some embodiments, the method further includes:

determining adjustment prompt information based on the volume expansion rate of the airbag within the T seconds; and

displaying the adjustment prompt information in the first interface.

For example, Table 1 shows a correspondence between the volume expansion rate and the adjustment prompt information.

**Table 1 Correspondence table between a volume expansion rate and adjustment prompt information**

| Volume expansion rate | Adjustment prompt information |
|---|---|
| Volume expansion rate$\geq$4 | Prompt to tighten by three positions |
| 3$\leq$Volume expansion rate<4 | Prompt to tighten by two positions |
| 2$\leq$Volume expansion rate<3 | Prompt to tighten by two positions |
| 1.3$\leq$Volume expansion rate$\leq$1.5 | Prompt to loosen by one position |
| 1.1$\leq$Volume expansion rate<1.3 | Prompt to loosen by two positions |

For example, as shown in FIG. 2(e), when determining that the smartwatch is worn too tight, the smartwatch may display, in the interface 203, the adjustment prompt information: "Loosen the watch strap by one position".

In this embodiment of this application, the wearable device may display the adjustment prompt information based on the volume expansion rate. The adjustment prompt information may prompt the user for an adjustment manner and an adjustment amplitude, so that the user can perform more accurate tightness adjustment. This ensures accuracy of a physiological parameter measurement result, without adding hardware, and without incurring additional costs.

It should be noted that, the foregoing embodiment uses an example in which the wearable device can detect that the wearable device is worn too loose and detect that the wearable device is worn too tight. However, this is not limited in this embodiment of this application. In some other embodiments, the wearable device may detect only that the wearable device is worn too loose by using the foregoing method, or may detect only that the wearable device is worn too tight by using the foregoing method. It is not difficult to understand that when the wearable device detects only that the wearable device is worn too loose, or may detect only that the wearable device is worn too tight, only one threshold may be specified to determine that the wearable device is worn too loose or too tight.

For example, the specified threshold is the first threshold, and when the volume expansion rate is greater than the first threshold, it is determined that the device is worn too loose.

For another example, the specified threshold is the second threshold, and when the volume expansion rate is less than the second threshold, it is determined that the device is worn too tight.

The foregoing mainly describes, from a perspective of the wearable device, the wearing detection method for the wearable device provided in embodiments of this application. It may be understood that, to implement the foregoing functions, the wearable device includes a corresponding hardware structure and/or a corresponding software module for performing each function. A person of ordinary skill in the art should easily be aware that, in combination with algorithms and steps in the examples described in embodiments disclosed in this specification, this application can be implemented by hardware or a combination of hardware and computer software. Whether a specific function is performed by hardware or hardware driven by computer software depends on particular applications and design constraint conditions of the technical solutions. A person skilled in the art may use different methods to implement the described functions for each particular application, but it should not be considered that the implementation goes beyond the scope of this application.

In embodiments of this application, functional modules (or units) of processors in the wearable device may be obtained through division based on the foregoing method examples. For example, each functional module (or unit) may be obtained through division based on each corresponding function, or two or more functions may be integrated into one processing module (or unit). The integrated module (or unit) may be implemented in a form of hardware, or may be implemented in a form of a software functional module (or unit). It should be noted that, in embodiments of this application, division into the modules (or units) is an example and is merely logical function division, and may be other division in an actual implementation.

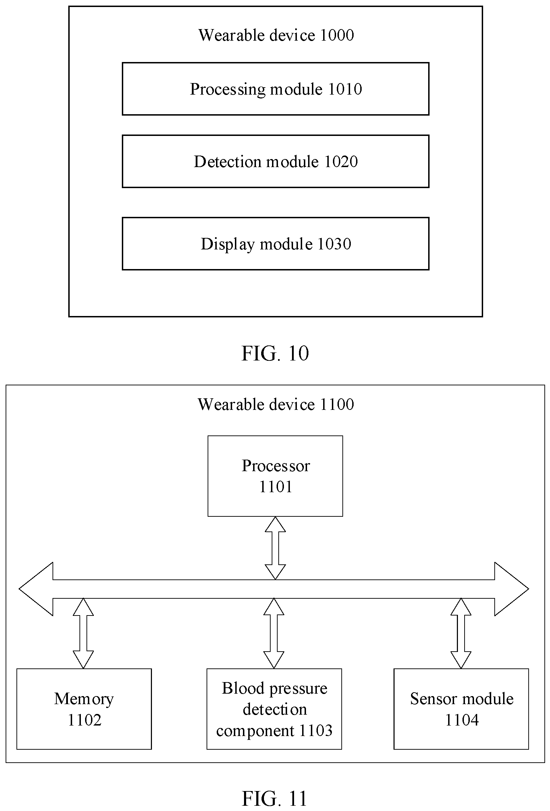

When each functional module (or unit) corresponding to each function is obtained through division, FIG. 10 is a block diagram of a wearable device 1000 according to an embodiment of this application. As shown in FIG. 10, the wearable device 1000 includes a processing module 1010, a detection module 1020, and a display module 1030.

The processing module 1010 is configured to: when it is determined that a preset condition is met, inflate an airbag.

The detection module 1020 is configured to detect a volume expansion rate of the airbag within T seconds.

The processing module 1010 is further configured to determine a wearing detection result of the wearable device based on the volume expansion rate of the airbag within the T seconds.

The display module 1030 is configured to display a first interface, where the first interface includes the wearing detection result.

In some embodiments, when the volume expansion rate is greater than the first threshold, it is determined that the device is worn too loose.

In some embodiments, when the volume expansion rate is less than a second threshold, it is determined that the device is worn too tight.

In some embodiments, when the volume expansion rate is greater than or equal to the second threshold and less than or equal to the first threshold, it is determined that the device is normally worn.

Optionally, in some embodiments, the detection module 1020 is further configured to detect performance of an air pump before the processing module 1010 inflates the airbag.

The processing module 1010 is further configured to determine a first weight parameter based on the performance of the air pump.

The processing module 1010 is specifically configured to determine the wearing detection result of the wearable device based on the volume expansion rate of the airbag within T seconds and the first weight parameter.

Optionally, in some embodiments, the detection module 1020 is further configured to detect a wearing posture of a user before the processing module 1010 inflates the airbag.

The processing module 1010 is further configured to determine a second weight parameter based on the wearing posture.

The processing module 1010 is specifically configured to: determine the wearing detection result of the wearable device based on the volume expansion rate of the airbag within the T seconds, the first weight parameter, and the second weight parameter, or determine the wearing detection result of the wearable device based on the volume expansion rate of the airbag within the T seconds and the second weight parameter.

Optionally, in some embodiments, the detection module 1020 is specifically configured to: inflate the airbag; when a pressure of the airbag reaches a first pressure, perform deflation; and determine the performance of the air pump based on a deflation rate.

Optionally, in some embodiments, the detection module 1020 is specifically configured to: inflate the airbag, where a duty cycle of the airbag is a first duty cycle; and when a pressure of the airbag reaches a second pressure, determine an inflation time period, and determine the performance of the air pump based on the inflation time period.

Optionally, in some embodiments, the preset condition is an operation of triggering physiological parameter measurement by the user.

Optionally, in some embodiments, the preset condition is an operation of triggering wearable device wearing detection by the user.

Optionally, in some embodiments, the preset condition is that a difference between a measured physiological parameter value and an average value of physiological parameter values measured in a first time period is greater than a third threshold.

Optionally, in some embodiments, the processing module 1010 is further configured to determine adjustment prompt information based on the volume expansion rate of the airbag within the T seconds.

The display module 1030 is further configured to display the adjustment prompt information in the first interface.

It should be understood that implementation principles and technical effects of FIG. 10 are similar to those in the foregoing method embodiment, and details are not described herein again.

FIG. 11 shows a diagram of a structure of a wearable device 1100 according to an embodiment of this application. As shown in FIG. 11, the wearable device 1100 includes a processor 1101, a memory 1102, a blood pressure detection component 1103, and a sensor module 1104. The sensor module 1104 includes one or more sensors configured to implement the foregoing method embodiments. The processor 1101, the memory 1102, the blood pressure detection component 1103, and the sensor module 1104 communicate with each other by using an internal connection path, to transmit a control signal and/or a data signal. In a possible design, the processor 1101 and the memory 1102 may be implemented by using a chip. The processor 1101 and the memory 1102 may be implemented in a same chip, or may be respectively implemented in different chips, or any two of the functions are combined and implemented in one chip. The memory 1102 may store program code, and the processor 1101 invokes the program code stored in the memory 1102, so that the wearable device implements the technical solutions in the foregoing embodiments.

An embodiment of this application provides a computer program product. When the computer program product runs on a wearable device, the wearable device is enabled to perform the technical solutions in the foregoing embodiments. Implementation principles and technical effects thereof are similar to those of the foregoing method embodiments, and details are not described herein again.

An embodiment of this application provides a readable storage medium. The readable storage medium includes instructions. When the instructions are run on a wearable device, the wearable device is enabled to perform the technical solutions in the foregoing embodiments. Implementation principles and technical effects thereof are similar. Details are not described herein again.

An embodiment of this application provides a chip. The chip is configured to execute instructions. When the chip runs, the technical solutions in the foregoing embodiments are performed. Implementation principles and technical effects thereof are similar. Details are not described herein again.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of embodiments of this application.

It may be clearly understood by a person skilled in the art that, for the purpose of convenient and brief description, for a detailed working process of the foregoing system, apparatus, and unit, refer to a corresponding process in the foregoing method embodiment. Details are not described herein again.

In the several embodiments provided in this application, it should be understood that the disclosed system, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of this application may be integrated into one processing unit, each of the units may exist alone physically, or two or more units may be integrated into one unit.

When the functions are implemented in the form of a software functional unit and sold or used as an independent product, the functions may be stored in a computer-readable storage medium. Based on such an understanding, the technical solutions of embodiments of this application essentially, or the part contributing to the conventional technology, or a part of the technical solutions may be implemented in a form of a software product. The computer software product is stored in a storage medium, and includes several instructions for instructing a computer device (which may be a personal computer, a server, a network device, or the like) to perform all or some of the steps of the methods described in embodiments of this application. The foregoing storage medium includes any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of embodiments of this application. However, the protection scope of embodiments of this application is not limited thereto. Any change or replacement readily figured out by a person skilled in the art within the technical scope disclosed in embodiments of this application shall fall within the protection scope of embodiments of this application. Therefore, the protection scope of embodiments of this application shall be subject to the protection scope of the claims.

**Claims**

1. A wearing detection method for a wearable device, wherein the wearable device comprises a main body, a wrist strap, and a blood pressure detection component, the blood pressure detection component comprises an airbag and an air pump, and the method comprises:

   detecting that a preset condition is met, and inflating the airbag, wherein an inflation time period is T seconds, and T>0;
   detecting a volume expansion rate of the airbag within the T seconds;
   determining a wearing detection result of the wearable device based on the volume expansion rate of the airbag within the T seconds; and
   displaying a first interface, wherein the first interface comprises the wearing detection result.

2. The method according to claim 1, wherein before the determining that a preset condition is met, and inflating the airbag, the method further comprises:

   detecting performance of the air pump, and determining a first weight parameter based on the performance of the air pump; and
   the determining a wearing detection result of the wearable device based on the volume expansion rate of the airbag within the T seconds comprises:
   determining the wearing detection result of the wearable device based on the volume expansion rate of the airbag within the T seconds and the first weight parameter.

3. The method according to claim 2, wherein before the determining that a preset condition is met, and inflating the airbag, the method further comprises:

    determining a wearing posture of a user, and determining a second weight parameter based on the wearing posture; and
    the determining a wearing detection result of the wearable device based on the volume expansion rate of the airbag within the T seconds comprises:
    determining the wearing detection result of the wearable device based on the volume expansion rate of the airbag within the T seconds, the first weight parameter, and the second weight parameter.

4. The method according to claim 2 or 3, wherein the detecting performance of the air pump comprises:

    inflating the airbag;
    when a pressure of the airbag reaches a first pressure, performing deflation; and
    determining the performance of the air pump based on a deflation rate.

5. The method according to claim 2 or 3, wherein the detecting performance of the air pump comprises:

    inflating the airbag, wherein a duty cycle of the air pump is a first duty cycle;
    when a pressure of the airbag reaches a second pressure, determining an inflation time period; and
    determining the performance of the air pump based on the inflation time period.

6. The method according to claim 1, wherein before the determining that a preset condition is met, and inflating the airbag, the method further comprises:

    determining a wearing posture of a user, and determining a second weight parameter based on the wearing posture; and
    the determining a wearing detection result of the wearable device based on the volume expansion rate of the airbag within the T seconds comprises:
    determining the wearing detection result of the wearable device based on the volume expansion rate of the airbag within the T seconds and the second weight parameter.

7. The method according to any one of claims 1 to 6, wherein the detecting a volume expansion rate of the airbag within the T seconds comprises:

    determining the volume expansion rate of the airbag within the T seconds according to the following formula:

$$\varepsilon_t = \frac{P_0}{P_t}\left(1 + \frac{W'(t)}{n'_0}\right)$$

$$\varepsilon = \frac{\sum_{t=0}^{T} \varepsilon_t}{T}$$

    $P_0$ is a pressure inside the airbag when the airbag is not inflated, $P_t$ is a pressure inside the airbag at a moment t when the airbag is inflated, W'(t) is a difference between an inflation rate and a deflation rate at the moment t, $n'_0$ is an amount of substance of air, and $\varepsilon$ is the volume expansion rate of the airbag within the T seconds.

8. The method according to any one of claims 1 to 7, wherein the method further comprises:

    determining adjustment prompt information based on the volume expansion rate of the airbag within the T seconds; and
    displaying the adjustment prompt information in the first interface.

9. The method according to any one of claims 1 to 8, wherein the determining a wearing detection result of the wearable device based on the volume expansion rate of the airbag within the T seconds comprises:

when the volume expansion rate of the airbag within the T seconds is greater than a first threshold, determining that the wearable device is worn too loose;

when the volume expansion rate of the airbag within the T seconds is less than a second threshold, determining that the wearable device is worn too tight; or

when the volume expansion rate of the airbag within the T seconds is greater than or equal to the second threshold and is less than or equal to the first threshold, determining that the wearable device is normally worn.

10. The method according to any one of claims 1 to 9, wherein the detecting that a preset condition is met comprises:

detecting an operation of triggering physiological parameter measurement by the user; and/or

detecting an operation of triggering wearable device wearing detection by the user; and/or

detecting that a difference between a measured physiological parameter value and an average value of physiological parameter values measured in a first time period is greater than a third threshold.

11. A wearable device, comprising one or more processors, one or more memories, and a blood pressure detection component, wherein the one or more memories store one or more computer programs, the one or more computer programs comprise instructions, and when the instructions are executed by the one or more processors, the method according to any one of claims 1 to 10 is performed.

12. A chip, wherein the chip comprises a processor and a communication interface, the communication interface is configured to: receive a signal, and transmit the signal to the processor, and the processor processes the signal, to enable the method according to any one of claims 1 to 10 to be performed.

13. A computer-readable storage medium, wherein the computer-readable storage medium stores computer instructions, and when the computer instructions are run on a computer, the method according to any one of claims 1 to 10 is performed.

FIG. 1

Clock    Gallery    Music

Settings    Recorder    AppGallery

A

201

202

FIG. 2(a)

The smartwatch is currently worn too loose. Adjust the smartwatch in time

Wearing redetection

203

204

FIG. 2(b)

EP 4 527 289 A1

The smartwatch is currently worn too tight. Adjust the smartwatch in time

203

204

Wearing redetection

FIG. 2(c)

The smartwatch is currently normally worn. No adjustment is required

203

FIG. 2(d)

FIG. 2(f)

FIG. 2(e)

Clock    Gallery    Music

Settings    Recorder    AppGallery

301

302

Blood pressure
measurement

FIG. 3(a)

The smartwatch is currently worn too
loose. Adjust the smartwatch in time, and
then perform blood pressure measurement

303

Wearing redetection

304

FIG. 3(b)

EP 4 527 289 A1

The smartwatch is currently worn too tight. Adjust the smartwatch in time, and then perform blood pressure measurement

303

304

Wearing redetection

FIG. 3(c)

Diastolic pressure: 75 mmHg
Systolic pressure: 125 mmHg

303

305

Blood pressure remeasurement

FIG. 3(d)

Clock    Gallery    Music

Settings    Recorder    AppGallery

401

402

Blood pressure
measurement

(a)

403

Perform wearing detection?

401

404    405

OK    Cancel

Blood pressure
measurement

(b)

FIG. 4

EP 4 527 289 A1

FIG. 5(a)

FIG. 5(b)

EP 4 527 289 A1

A current blood pressure measurement result is greatly different from a weekly average blood pressure. Perform wearing detection?

503

504

505

OK

Cancel

**FIG. 5(c)**

The smartwatch is currently worn too tight. Adjust the smartwatch in time, and then perform blood pressure measurement

506

507

Wearing redetection

508

Blood pressure remeasurement

**FIG. 5(d)**

S601: Detect that a preset condition is met, and inflate an airbag

S602: Detect a volume expansion rate of the airbag within T seconds

S603: Determine a wearing detection result of a wearable device based on the volume expansion rate of the airbag within the T seconds

S604: Display a first interface, where the first interface includes the wearing detection result

FIG. 6

S605: Detect performance of an air pump, and determine a first weight parameter based on the performance of the air pump

S601: Detect that a preset condition is met, and inflate an airbag

S602: Detect a volume expansion rate of the airbag within T seconds

S6031: Determine a wearing detection result of a wearable device based on the volume expansion rate of the airbag within the T seconds and the first weight parameter

S604: Display a first interface, where the first interface includes the wearing detection result

FIG. 7

S606: Detect a wearing posture of a user, and determine a second weight parameter based on the wearing posture of the user

S601: Detect that a preset condition is met, and inflate an airbag

S602: Detect a volume expansion rate of the airbag within T seconds

S6032: Determine a wearing detection result of a wearable device based on the volume expansion rate of the airbag within the T seconds and the second weight parameter

S604: Display a first interface, where the first interface includes the wearing detection result

FIG. 8

S605: Detect performance of an air pump, and determine a first weight parameter based on the performance of the air pump

S606: Detect a wearing posture of a user, and determine a second weight parameter based on the wearing posture of the user

S601: Detect that a preset condition is met, and inflate an airbag

S602: Detect a volume expansion rate of the airbag within T seconds

S6033: Determine a wearing detection result of a wearable device based on the volume expansion rate of the airbag within the T seconds, the first weight parameter, and the second weight parameter

S604: Display a first interface, where the first interface includes the wearing detection result

FIG. 9

Wearable device 1000

Processing module 1010

Detection module 1020

Display module 1030

FIG. 10

Wearable device 1100

Processor
1101

Memory
1102

Blood pressure
detection
component 1103

Sensor module
1104

FIG. 11

| INTERNATIONAL SEARCH REPORT | International application No. |
|---|---|
| | **PCT/CN2024/100910** |

**A.    CLASSIFICATION OF SUBJECT MATTER**

A61B5/022(2006.01)i;  A61B5/00(2006.01)i

According to International Patent Classification (IPC) or to both national classification and IPC

**B.    FIELDS SEARCHED**

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT; ENTXTC; ENTXT; VEN; CJFD; CNKI; 百度学术, BAIDU SCHOLAR; IEEE: 华为, 气囊, 气袋, 袖带, 袖套, 膨胀, 膨大, 体积, 容积, 速率, 速度, 变化率, 快慢, 佩戴, 穿戴, 接触, 松, 紧, 压力, 充气, 姿势, 姿态, balloon, cuff, expansion, volume, rate, speed, fast, slow, wear, tight, loose

**C.    DOCUMENTS CONSIDERED TO BE RELEVANT**

| Category* | Citation of document, with indication, where appropriate, of the relevant passages | Relevant to claim No. |
|---|---|---|
| A | CN 112568884 A (HUAWEI DEVICE CO., LTD.) 30 March 2021 (2021-03-30) description, paragraphs [0002]-[0176], and figures 1-12 | 1-13 |
| A | CN 113520307 A (HUAWEI TECHNOLOGIES CO., LTD.) 22 October 2021 (2021-10-22) entire document | 1-13 |
| A | CN 217186110 U (SHENZHEN KINGYIELD MEDICAL EQUIPMENT CO., LTD.) 16 August 2022 (2022-08-16) entire document | 1-13 |
| A | US 2014182352 A1 (GENERAL ELECTRIC COMPANY) 03 July 2014 (2014-07-03) entire document | 1-13 |
| A | US 2021145450 A1 (LEGIONARIUS LLC) 20 May 2021 (2021-05-20) entire document | 1-13 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 August 2024** | **23 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT
Information on patent family members

International application No.

**PCT/CN2024/100910**

| Patent document cited in search report | | | Publication date (day/month/year) | Patent family member(s) | Publication date (day/month/year) |
|---|---|---|---|---|---|
| CN | 112568884 | A | 30 March 2021 | None | |
| CN | 113520307 | A | 22 October 2021 | None | |
| CN | 217186110 | U | 16 August 2022 | None | |
| US | 2014182352 | A1 | 03 July 2014 | None | |
| US | 2021145450 | A1 | 20 May 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311010001X